# EUROPEAN PATENT APPLICATION

(11) **EP 4 332 884 A1**
(43) Date of publication of application: **06.03.2024**
(21) Application number: 22193692.5
(22) Date of filing: 02.09.2022
(51) Int. Cl.: G06T 7/00, G06T 7/13

(54) **DETECTING ANATOMICAL ABNORMALITIES IN 2D MEDICAL IMAGES**

(71) Applicant: Koninklijke Philips N.V., 5656 AG Eindhoven (NL)
(72) Inventor: VON BERG, Jens, Eindhoven (NL); YOUNG, Stewart Matthew, Eindhoven (NL); HERTGERS, Omar, 5656 AG Eindhoven (NL); BRUECK, Heiner Matthias, 5656 AG Eindhoven (NL); KROENKE-HILLE, Sven, Eindhoven (NL); BYSTROV, Daniel, Eindhoven (NL); GOOßEN, André, Eindhoven (NL); HARDER, Tim Philipp, Eindhoven (NL)
(74) Representative: Philips Intellectual Property & Standards

(57) **Abstract**

The invention relates to detecting anatomical abnormalities in medical images. In order to detect anatomical abnormalities, a computer-implemented method (100) and system are disclosed that detect 2D contours (130) of anatomical features in a medical image and compares these contours with predicted 2D contours (140) based on a 3D reference model in order to detect (150) anatomical abnormalities. This approach may improve accuracy of anatomical abnormality detection, thereby cutting time in a medical facility and potentially improving operator experiences and patient outcomes.

## Description

### FIELD OF THE INVENTION

The invention relates to a computer-implemented method, a computer program product, and a system configured for detecting anatomical abnormalities in 2D medical images.

### BACKGROUND OF THE INVENTION

Medical images are often used for diagnostic purposes. For example, medical images are examined by human observers for anatomical abnormalities. Examples of such abnormalities include bone fractures and tumorous tissue, which may be identified in X-ray, ultrasound, CT (Computer Tomography) or other medical images.

The inspection of medical images by human observers however is time consuming and prone to errors, depending on the expertise, experience, time pressure and exhaustion of the human observer. This is particularly the case in the hectic environment of a busy clinic and when the anatomical abnormalities are not easily discernible.

### SUMMARY OF THE INVENTION

It is, *inter alia*, an object of the invention to provide a computer-implemented anatomical abnormality detection in medical imaging. The invention is defined by the independent claims. Advantageous embodiments are defined in the dependent claims.

An aspect of the present invention provides a computer-implemented method for detecting an anatomical abnormality in a 2D medical image. The method comprises
- obtaining the 2D medical image;
- detecting a 2D contour of an anatomical feature;
- obtaining a 3D model representative of the anatomical feature;
- predicting a 2D contour of the anatomical feature based on the 3D model;
- detecting, based on the detected 2D contour and the predicted 2D contour, the anatomical abnormality. In this manner, 2D contours detected in an X-ray image may be compared to a known reference contour obtained from a 3D model, such as to detect and identify differences, which may be anatomical abnormalities. This approach may improve accuracy of anatomical abnormality detection, thereby cutting time in a medical facility and potentially improving operator experiences and patient outcomes.

The step of detecting a 2D contour may further include segmenting the 2D medical image. For example, individual pixels of an acquired medical image may be labeled as "bone/no bone", such as to identify bones in the image, and thereby detecting the 2D contours. Alternatively, the step of detecting a 2D contour may include an end-to-end trained machine learning algorithm. Additionally, the step of detecting a 2D contour may include identifying an anatomical feature. For example, identifying that the 2D contour is that of one or more bones of an ankle joint or a wrist. This approach may further be used to optimize the workflow of any further processing or to provide guidance to the user or machine for further steps.

The method may further include classifying the anatomical abnormality. The step of classifying the anatomical abnormality may further be performed by a machine learning algorithm. In this manner, the output of the computer-implemented method is not binary, e.g. anatomical abnormality detected or not detected, but provides the user insight into the classification of said abnormality. For example, whether it is severe or not, and/or whether it is a specific type of bone fracture and/or ligament rupture.

According to embodiments of the invention, the 3D model is obtained based on
- the 2D medical image,
- the anatomical feature, and/or
- a user input.

The 3D model may further be any one of:
- a computer aided design model; and
- a reference model constructed from 3D medical images of the anatomical feature in at least one pose.

In some examples, the method further comprises
- generating a difference between
- the detected 2D contour and
- the predicted 2D contour.

Additionally, a difference map may be generated. Said difference map containing information suitable to be displayed on a display.

According to an embodiment of the invention, the step of predicting a 2D contour of the anatomical feature based on the 3D model comprises:
- estimating a pose of the anatomical feature from the 2D medical image;
- adjusting the 3D model based on the estimated pose;
- generating a 2D projection from the pose adjusted 3D model, and
- predicting a 2D contour of the anatomical feature from the 2D projection. In other words, based on the detected 2D contours, a viewing position and pose of the imaged object may be estimated. A 3D model may then be rotated and translated to match the position of the imaged anatomical feature with respect to a virtual imaging system, and the 3D model may be adjusted by rotating its own articulations, e.g. varying the flexion of an ankle joint, to match the pose of the imaged anatomical feature. Thereby creating a healthy reference model with the same viewing and pose parameters as the imaged object, which may be used to predict 2D reference contours.

In some examples, the anatomical feature is any one of
- bone tissue, and
- ligament tissue.

In some examples, the anatomical abnormality is any one of
- bone fracture, and
- ligament rupture.

In some examples, the step of classifying the anatomical abnormality classifies the anatomical abnormality according to any one of
- Weber classification,
- Pauwels classification,
- Smith and Colles classification, and
- Salter-Harris classification.

In some examples, the medical image is an X-ray image.

Another aspect of the invention provides a computer program product comprising instructions for enabling a processor to carry out an embodiment of the above method.

Yest another aspect of the invention provides a system for detecting an anatomical abnormality in a 2D medical image. The system comprising a processor configured to
- obtain the 2D medical image;
- detect a 2D contour of an anatomical feature;
- obtain a 3D model representative of the anatomical feature;
- predict a 2D contour of the anatomical feature based on the 3D model;
- detect, based on the detected 2D contour and the predicted 2D contour, the anatomical abnormality.

In an example, the system may further comprise an X-ray source and detector configured for taking an X-ray image.

These and other aspects of the invention will be apparent from and elucidated with reference to the embodiments described hereinafter.

### BRIEF DESCRIPTION OF THE DRAWINGS

Fig. 1 shows a flow chart in accordance with an embodiment of the invention;
Fig. 2A shows a medical image in accordance with an embodiment of the invention;
Fig. 2B shows detected contours of anatomical features in a medical image in accordance with an embodiment of the invention;
Fig. 2C shows predicted contours of anatomical features in a medical image in accordance with an embodiment of the invention;
Fig. 3 shows pose and viewing parameters of an imaged object in accordance with an embodiment of the invention;
Fig. 4 shows an articulated 3D model of an ankle in accordance with an embodiment of the invention;
Fig. 5A shows a classification of ankle fractures in accordance with an embodiment of the invention;
Fig. 5B shows a classification of femoral neck fractures in accordance with an embodiment of the invention;
Fig. 5C shows a classification of fractures through the epiphyseal or growth plate of a bone in accordance with an embodiment of the invention; and
Fig. 6 shows a processor circuit in accordance with an embodiment of the invention.

### DESCRIPTION OF EMBODIMENTS

The invention will be described herein with reference to the Figures. It should be understood that the description and specific examples provided, while indicating exemplary embodiments, are intended for purposes of illustration only, and not intended to limit the scope of the invention. It should also be understood that the Figures are mere schematics and not drawn to scale. It should also be understood that the same reference numerals are used throughout the Figures to indicate the same or similar parts.

The invention provides a computer-implemented method of analyzing and assessing 2D (two-dimensional) medical images with the purpose of identifying anatomical abnormalities.

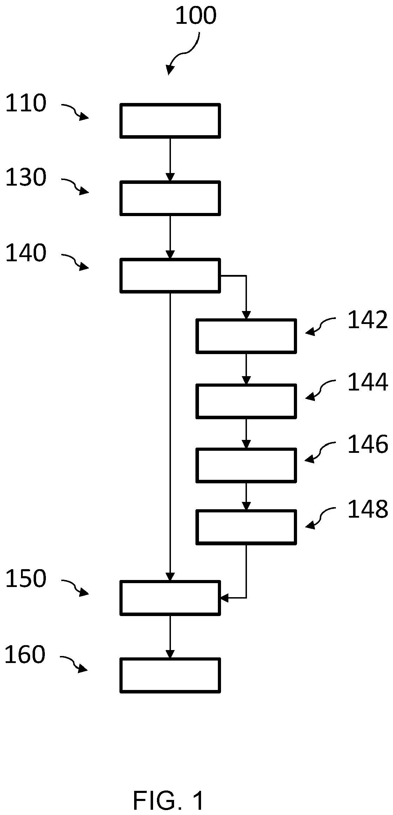

Fig. 1 shows an exemplary flow chart according to an embodiment of a method 100 according to the invention that comprises the following steps:
In step 110, a 2D medical image 210 is obtained. The 2D medical image may be any type of medical image, including but not limited to for example an X-ray image, a CT (computer tomography) image, an ultrasound image, an MR (magnetic resonance) image, or any other type of medical image.

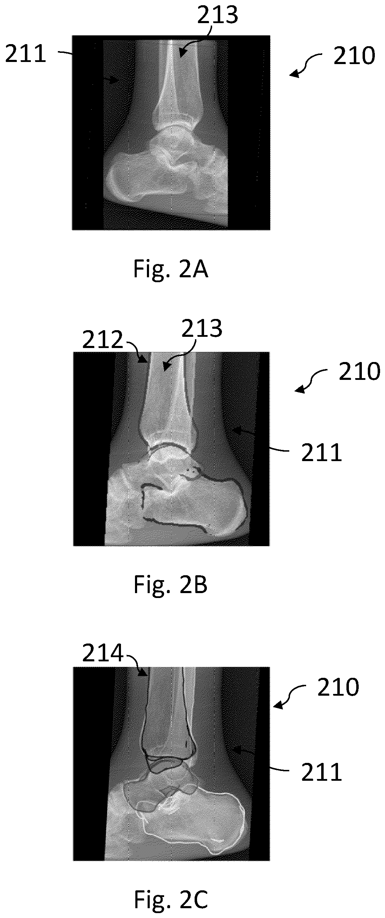

An exemplary medical image 210 as envisaged in this invention is the 2D X-ray image of an ankle joint 211 shown in Fig. 2A.

In step 120, a 2D contour of an anatomical feature is detected. This may for example include a segmentation of the 2D medical image or an end-to-end machine learning approach.

Broadly, segmentation may be understood as the task of classifying an image per pixel into labels. Alternatively, the classification is per larger image regions as whole, such as contours or surfaces. The labels represent a semantics of the respective pixel or image region. In this manner, for example a bone pixel, that is, a pixel having the label "bone", can be identified in the classification operation. The pixel is then thought to represent bone tissue, and so forth for other tissue types. Footprints of organs in the imagery can thus be identified by segmentation. In an alternative embodiment, the segmentation may be performed using a neural network or a convolutional neural network, such as disclosed in WO 2022/084074. Algorithms including prior shape knowledge as disclosed therein are also envisaged by this invention.

From an image segmentation, a segmented image is typically obtained, in which each pixel and/or region has a specific label assigned to it. This label may be binary "bone/no bone" or multilevel "bone/ligament tissue/no bone/... ". According to an embodiment of this invention, the parts in the medical image corresponding with an anatomical feature of interest, for example "bone" or more specifically, "tibia/fibula/calcaneus/talus", may be selected to generate a 2D contour 212 as shown in Fig. 2B.

In step 130, a 3D (three-dimensional) model 330 representative of the imaged object 211 or the identified anatomical feature 213 is obtained. The model may be selected from a list of 3D models based on at least one of:
- the medical image 210 obtained in step 110,
- the identified anatomical feature in step 120, and
- a user input.

For example, the image 210 obtained in step 110 may be annotated to indicate that the image is of an ankle joint. In another example, the identified anatomical feature or combination of identified anatomical features may indicate that the image contains at least an ankle joint. For example, a DICOM tag may be associated with the anatomical feature being imaged, which may be used to identify the 3D model. Yet in another example, a user of a computing system executing the method 100, may select the 3D model from a list of models. The user selection may be from the entirety of all 3D models, or from a reduced set of articulated 3D models, which may be pre-selected based on the 2D medical image and/or identified anatomical features in the 2D medical image. In another example, the 3D model may be selected based on context information at the acquisition place, e.g. Radiology department in a hospital, based on for example user diagnostic request data and/or radiological standard operation procedure for a requested examination procedure. For example, before the image acquisition, a user or operator may indicate a desire to take an ankle joint X-ray image, which may determine the relevant 3D model, or create a pre-selection of relevant 3D models. The 3D model may be articulated such as to be able to account for different poses of the imaged object, for example the 3D model may be capable of reproducing a specific flexion of an ankle joint.

A representative 3D model may be a computer aided design (CAD) model, created with any CAD software, or a reference model extracted from 3D imaging using known techniques. For example, a sequence of MR images of an ankle joint of a healthy subject may be obtained at varying flexion angles. One image may then be segmented manually or automatically according to known techniques. The segmented image may then be used to create a surface-model using for example a triangular mesh. For each triangle in the mesh, features for model-adaption may then be trained using for example the image intensity, and the resulting model may be used to delineate corresponding bone surfaces in the other MR images. This procedure may yield a 3D model per MR image. The pose in each image and/or corresponding 3D model may further be determined by for example measuring the angle between the main axis of the tibia and that of the calcaneus for flexion. Smooth interpolation of the surface models between the varying poses recorded in the MR images, may enable the mesh constellation to be estimated at any pose therebetween.

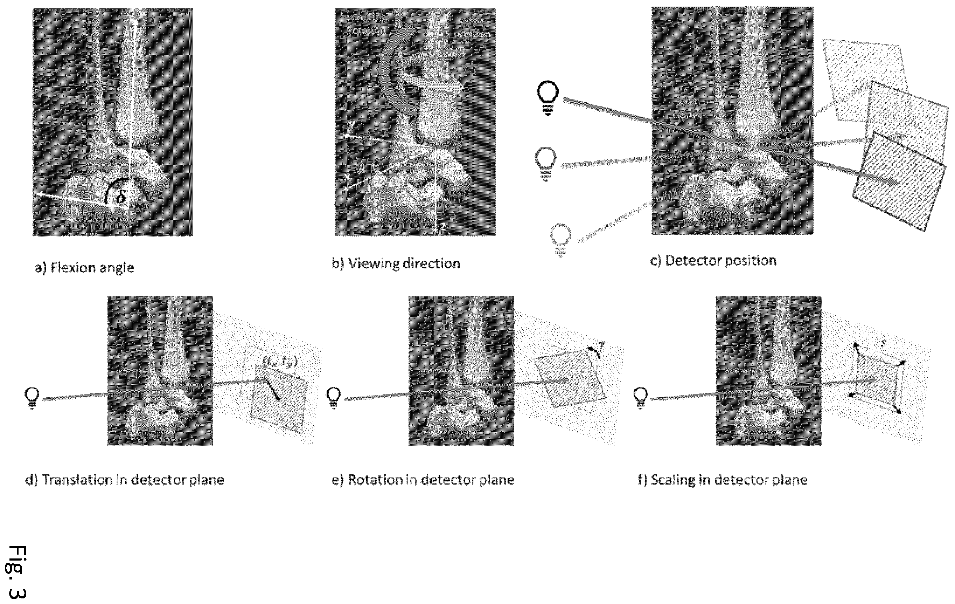

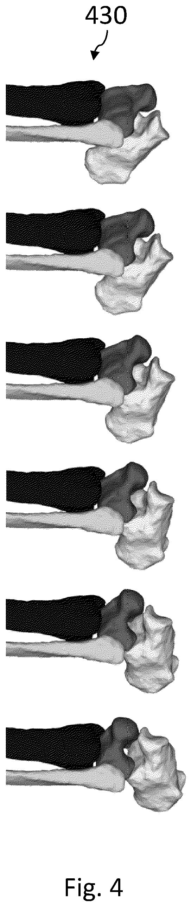

A 3D model 430 as envisaged by the invention is shown in Fig. 4. The 3D model may be articulated and may be modified such as to fit any pose encountered in a 2D medical image. For example, through the image sequence in Fig. 3 the calcaneus is shown to rotate with respect to the tibia from 80 degrees to 135 degrees flexion.

In step 140, a 2D contour of the anatomical features in the medical image is predicted from a 3D model. According to an exemplary implementation, the contour may be predicted according to the following steps:
- estimating 142, from the 2D medical image, viewing parameters and/or the pose of the imaged object,
- modifying or adjusting 144 the 3D model according to the estimated viewing parameters and/or pose,
- creating 146 a 2D projection from the viewing and/or pose adjusted 3D model, wherein the projection is representative of the 2D medical image 210, and
- generating 148 a 2D contour 214 of the anatomical features shown in the 2D projection of the 3D model.

The viewing parameters and pose as referred to in step 142, may be, as shown in Fig. 3 for an ankle joint, any one of for example:
- a flexion angle δ between the tibia and the calcaneus;
- a viewing direction, including azimuthal and polar rotation; and
- a detector position, translation, rotation and scaling in the case of X-ray images.

This list is not to be interpreted as exhaustive, and other viewing and/or pose parameters are also envisaged by the invention.

The pose and/or viewing parameters may be estimated in step 142 according to S. Krönke et al. 2022 "CNN-based pose estimation for assessing quality of ankle-joint X-ray images".

For example, a meshed 3D model of an ankle joint may be sampled at random flexion angles and viewing directions, and projections may be generated by identifying the triangles of the mesh in the 3D model which are traversed by the X-ray beam approximately tangentially for the chosen viewing direction, projecting their edges onto the virtual detector and semantically grouping and discretizing them on a detector pixel-matrix. The resulting set of discretized contours in the projections forms the input for a pose-estimation network. Such a pose-estimation network may be a standard regression network involving ReLU (Rectified Linear Unit) activation functions with alternating stride-1 and stride-2 convolutional blocks and fully connected layers. A network trained according to the above principle may then be used to predict the pose of detected 2D contours. For example, 2D contours of an imaged ankle joint will be fed into the neural network, which may, due to its training, provide the flexion angle and viewing direction of the imaged ankle joint.

The pose and/or viewing parameters estimated in step 142 may then be used as input to step 144 in modifying or adjusting the 3D model to reproduce the respective pose and/or viewing parameters. For example, the flexion angle of the 3D ankle model 430 may be varied as shown in Fig. 4, where the angle between the tibia and calcaneus is changed from 80 degrees to 135 degrees.

After adjusting the 3D model to the estimated pose, a 2D projection is determined in a step 146 by for example identifying the triangles of the mesh in the 3D model which are traversed by an X-ray beam approximately tangentially for the chosen viewing direction, projecting their edges onto a virtual detector and semantically grouping and discretizing them on a detector pixel-matrix, thereby also directly providing the predicted 2D contours of step 148. Fig. 2C shows exemplary 2D contours 214 as predicted in step 140, 142-148 from a 3D model of an ankle joint.

In an optional step, a difference between the 2D contours generated based on the 2D medical image and the 2D contours generated based on the articulated 3D model may be determined. This difference may be calculated based on for example a pointwise subtraction or pixel-by-pixel subtraction. In some implementations, a difference map may be created, wherein said difference map may also be visualized on a user interface (not shown).

In step 150, an anatomical abnormality is detected based on the 2D contours respectively detected in step 120 and predicted in step 140. The anatomical abnormality may be detected by visual inspection of for example a difference map shown on a user interface (not shown). In another exemplary embodiment, the anatomical abnormality may be detected automatically. For example, based on whether a difference or a deviation between (i) the 2D contours generated based on the 2D medical image and (ii) the 2D contours generated based on the articulated 3D model exceeds a particular threshold. In yet another exemplary embodiment, a machine learning algorithm may be used to detect anatomical abnormalities. For example, 2D medical images may be annotated by a user or expert to identify anatomical abnormalities. These annotated images may also be processed according to steps 120 and 140 in order to detect 2D contours and predict 2D contours, wherein the detected 2D contours, predicted 2D contours and/or a difference between the detected and predicted 2D contours may be added to the annotated images to be used in the training process.

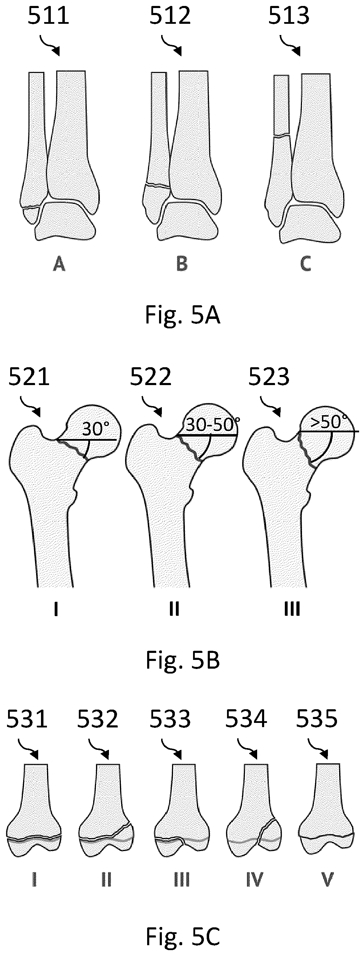

The identification of anatomical abnormalities may be binary, abnormality present or not present, or non-binary, in which case a probability may be provided that an abnormality is present. In an advantageous embodiment, the anatomical abnormalities may also be classified in an optional step 160 based on size, location, or any other form of classification, to indicate a level of severity. In other words, the anatomical abnormality may be classified into a plurality of categories. Fig. 5A, discussed in more detail below, shows for example a classification of ankle joint fractures, and Fig. 5B, also discussed in more detail below, shows for example a classification of femoral neck fracture classification. According to an embodiment of the present disclosure, the classification of the anatomical abnormality is performed with a trained machine learning algorithm. For example, a classification network may be trained with annotated data such as in Fig. 5A-5C and using the detected 2D contours 212 and predicted 2D contours 214 as inputs.

For the training of classification machine learning model, annotated medical images are provided. For example, X-ray images of ankle joints with annotation indicating whether a fracture is present and the classification of said fracture according to for example the Weber classification of Fig. 5A. The annotations may be performed by an expert such as a clinician, radiologist, researcher or any other qualified person. As an example, an annotated image may be the ankle joint X-ray image 210 with an associated label of no fracture. Another example may be an ankle joint X-ray image with a fracture and corresponding label identifying the classification of said fracture. For example, whether the fracture is a type A, B or C fracture in the Weber classification.

In the same manner as images to be analyzed through the method 100, annotated images used for training also undergo steps 120 and 140 of method 100 in order to
- detect 2D contours of an anatomical feature; and
- predict 2D contours of the anatomical feature from a healthy reference, for example from a 3D model obtained in step 130.

The detected 2D contours and predicted 2D contours may then form the input to a machine learning model, and the annotation of the training image the output. As usual, the training is performed with a subset of the training images and the corresponding annotations, and the remainder of the training images and corresponding annotations is used to evaluate the trained model. The training is stopped when a predetermined criterion is reached, such as to ensure that the model has been trained well enough but not over-trained. In some implementations, also noise may manually or automatically be added to the training data to avoid over-fitting.

It is understood by a person skilled in the art that while method 100 was presented in a specific order, the order of the steps may vary, additional steps may be added in between, and/or steps may be removed.

It is furthermore understood that the individual steps in the method 100 may be performed in real time, e.g., during an examination procedure, or thereafter. For example, a 2D medical image may be obtained in real time and analyzed according to method 100 during the image acquisition procedure, or the 2D medical image may be first obtained during an acquisition step and only be analyzed upon request of an operator and/or expert. The method 100 may thus be implemented within an image acquisition system or outside of it in a separate computing unit. In another embodiment the method 100 may also be carried out in a cloud.

Pre-defined classifications as envisaged by the invention are disclosed in Fig. 5A-5C. The classifications in these Figures are merely illustrative and in no way limiting of the idea of the invention.

Fig. 5A displays the Weber classification for ankle joints. In Fig. 5A, 3 different types of fractures are shown, type A 511, type B 512 and type C 513, which according to Wikipedia (https://en.wikipedia.org/wiki/Danis%E2%80%93Weber_classification) may be described as:
- Type A 511 - Fracture of the fibula distal to the syndesmosis (the connection between the distal ends of the tibia and fibula). Typical features are:
   - below level of the ankle joint
   - tibiofibular syndesmosis intact
   - deltoid ligament intact
   - medial malleolus occasionally fractured
   - usually stable: occasionally nonetheless requires an open reduction and internal fixation (ORIF) particularly if medial malleolus fractured.
- Type B 512 - Fracture of the fibula at the level of the syndesmosis. Typical features:
   - at the level of the ankle joint, extending superiorly and laterally up the fibula
   - tibiofibular syndesmosis intact or only partially torn, but no widening of the distal tibiofibular articulation
   - medial malleolus may be fractured or deltoid ligament may be torn
   - variable stability
- Type C 513 - Fracture of the fibula proximal to the syndesmosis. Typical features:
   - above the level of the ankle joint
   - tibiofibular syndesmosis disrupted with widening of the distal tibiofibular articulation
   - medial malleolus fracture or deltoid ligament injury present
   - unstable: requires ORIF

Fractures of type B 512 and C 513 imply a degree of damage to the syndesmosis itself (which may not be directly visualized on X-ray). They are inherently unstable and are more likely to require operative repair to achieve a good outcome. Type A fractures are usually stable and can be managed with simple measures, such as a plaster of Paris cast. The correct identification of an ankle fracture according to the Weber classification may thus improve the outcome and/or recovery of the imaged subject.

Fig. 5B displays the Pauwels classification for femoral neck fractures. These fractures are characterized by the fracturing angle. An increasing angle leads to a more unstable fracture and an increase in the shear stress at the fracture site. This shear leads to higher rates of nonunion. According to Wikipedia (https://en.wikipedia.org/wiki/Pauwel%27s_angle) the Pauwels classification, divides the fracture into type I 521, type II 522 and type III 523 fractures as follows:
- Type I 521 - fracture angle of less than 30 degrees
- Type II 522 - fracture angle between 30 and 50 degrees
- Type III 523 - fracture angle of more than 50 degrees.

Fig. 5C displays the Salter-Harris classification for fractures involving the epiphyseal or growth plate of a bone, and specifically the zone of provisional calcification. It is thus a form of child bone fracture, occurring in 15% of childhood long bone fractures. There are nine types of Salter-Harris fractures; types I 531 to V 535 as described by Robert B Salter and W Robert Harris in 1963 (shown in Fig. 5C), and the rarer types VI to IX which have been added subsequently (not shown or discussed here). According to Wikipedia (https://en.wikipedia.org/wiki/Salter%E2%80%93Harris_fracture), the first five types may be described as:
- Type I 531 - transverse fracture through the growth plate (also referred to as the "physis");
- Type II 532 - A fracture through the growth plate and the metaphysis, sparing the epiphysis: takes approximately 12-90 weeks or more in the spine to heal;
- Type III 533 - A fracture through growth plate and epiphysis, sparing the metaphysis;
- Type IV 534 - A fracture through all three elements of the bone, the growth plate, metaphysis, and epiphysis;
- Type V 535 - A compression fracture of the growth plate (resulting in a decrease in the perceived space between the epiphysis and metaphysis on x-ray).

The pre-defined classifications in Fig. 5A to 5C are provided as a reference to showcase the classification possible according to embodiments of the present invention. In particular, using pre-defined classes, allows an operator to see not only that a fracture may have occurred, but also what type of fracture may have occurred, and may thus improve the subsequent steps to improve the outcome and/or recovery of the imaged subject.

While pre-defined classifications with regards to bone and/or ligament fractures have been discussed above, other types of classifications including but not limited to for example cancer types and/or stages are also envisaged by this invention. For example, a segmented tumor may automatically be classified according to its size and/or location into a stage I, stage II, stage III or stage IV tumor.

The method 100 may further include an optional step 170 to provide treatment advice based on the identified anatomical abnormality and/or the classification result of the identified anatomical abnormality. For example, the treatment advice may be immediate surgery needed to a type C Weber classified ankle fracture, while for a hairline and/or stress fracture, rest and/or cast may be advised. The advice may be based on annotated data, for example, the classification images may also be annotated to provide a treatment advice according to the classifications as discussed above.

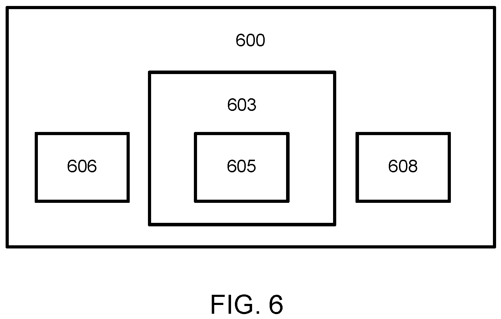

Fig. 6 is a schematic diagram of a processor circuit 600, according to aspects of the present disclosure. As shown, the processor circuit 600 may include a processor 606, a memory 603, and a communication module 608. These elements may be in direct or indirect communication with each other, for example via one or more buses.

The processor 606 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 606 may also be implemented as a combination of computing devices, e.g., a combination of a DSP and a microprocessor, a plurality of microprocessors, one or more microprocessors in conjunction with a DSP core, or any other such configuration. The processor 606 may also implement various deep learning networks, which may include a hardware or a software implementation. The processor 606 may additionally include a preprocessor in either hardware or software implementation.

The memory 603 as envisaged by the present disclosure may be any suitable storage device, such as a cache memory (e.g., a cache memory of the processor 606), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processor circuit. In an embodiment, the memory 603 may store instructions 605. The instructions 605 may include instructions that, when executed by a processor 606, cause the processor 606 to perform the operations described herein.

Instructions 605 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 205 may be in the form of an executable computer program or script. For example, routines, subroutines and/or functions may be defined in a programming language including but not limited to C, C++, C#, Pascal, BASIC, API calls, HTML, XHTML, XML, ASP scripts, JavaScript, FORTRAN, COBOL, Perl, Java, ADA, .NET, and the like. Instructions may also include instructions for machine learning and/or deep learning.

The communication module 608 can include any electronic circuitry and/or logic circuitry to facilitate direct or indirect communication of data between the processor circuit 600, and for example an external display (not shown) and/or an imaging device or system such as an X-ray imaging system. In that regard, the communication module 608 can be an input/output (I/O) device. The communication may be performed via any suitable means. For example, the communication means may be a wired link, such as a universal serial bus (USB) link or an Ethernet link. Alternatively, the communication means may be a wireless link, such as an ultra-wideband (UWB) link, an Institute of Electrical and Electronics Engineers (IEEE) 802.11 Wi-Fi link, or a Bluetooth link.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. Computer-implemented method (100) for detecting an anatomical abnormality in a 2D medical image (210), the method comprising:
- obtaining (110) the 2D medical image (210);
- detecting (120) a 2D contour (212) of an anatomical feature;
- obtaining (130) a 3D model (430) representative of the anatomical feature;
- predicting (140) a 2D contour (214) of the anatomical feature based on the 3D model (430);
- detecting (150), based on the detected 2D contour (212) and the predicted 2D contour (214), the anatomical abnormality.

2. The method (100) of claim 1, wherein the step of detecting the 2D contour (212) includes segmenting the 2D medical image (210).

3. The method (100) of any of the preceding claims, further comprising:
- classifying (160) the anatomical abnormality.

4. The method (100) of claim 3, wherein the step of classifying (160) the anatomical abnormality is performed by a machine learning algorithm.

5. The method (100) of any of the preceding claims, wherein the 3D model (430) is obtained based on
- the 2D medical image (210),
- the anatomical feature (213), and/or
- a user input.

6. The method (100) of any of the preceding claims, wherein the 3D model (430) is any one of:
- a computer aided design model; and
- a reference model constructed from 3D medical images of the anatomical feature in at least one pose.

7. The method (100) of any of the preceding claims, further comprising
- generating a difference between
- the detected 2D contour (212) and
- the predicted 2D contour (214).

8. The method (100) of any of the preceding claims, wherein the step (140) of predicting a 2D contour of the anatomical feature based on the 3D model comprises:
- estimating (142) a pose of the anatomical feature from the 2D medical image (210);
- adjusting (144) the 3D model (430) based on the estimated pose;
- generating (146) a 2D projection from the pose adjusted 3D model, and
- predicting (148) a 2D contour (214) of the anatomical feature (213) from the 2D projection.

9. The method (100) of any of the preceding claims, wherein the anatomical feature is any one of
- bone tissue, and
- ligament tissue.

10. The method (100) of any of the preceding claims, wherein the anatomical abnormality is any one of
- a bone fracture, and
- a ligament rupture.

11. The method (100) of any of claims 3 to 9, wherein the step of classifying the anatomical abnormality, classifies the anatomical abnormality based on any one of
- Weber classification,
- Pauwels classification,
- Smith and Colles classification, and
- Salter-Harris classification.

12. The method (100) of any of the preceding claims, further comprising
- generating (170) treatment advice, based on the detected anatomical abnormality.

13. The method (100) of any of the preceding claims, wherein the medical image is an X-ray image.

14. A computer program product comprising instructions for enabling a processor to carry out the method of any of the preceding claims.

15. A system for detecting an anatomical abnormality in a 2D medical image, the system comprising a processor (206) configured to carry out the method of any of the claims 1-13.
